(19) **Europäisches Patentamt / European Patent Office / Office européen des brevets**

(11) **EP 3 409 318 A1**

(12) # EUROPEAN PATENT APPLICATION
published in accordance with Art. 153(4) EPC

(43) Date of publication:
**05.12.2018 Bulletin 2018/49**

(21) Application number: **17744024.5**

(22) Date of filing: **17.01.2017**

(51) Int Cl.:
***A61M 37/00*** (2006.01)

(86) International application number:
**PCT/JP2017/001386**

(87) International publication number:
**WO 2017/130793 (03.08.2017 Gazette 2017/31)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **28.01.2016 JP 2016014320**
**10.08.2016 JP 2016157514**

(71) Applicant: **Ricoh Company, Ltd.**
**Tokyo 143-8555 (JP)**

(72) Inventors:
• **YAMABE, Atsumi**
  **Tokyo 143-8555 (JP)**
• **KANEMATSU, Toshihiro**
  **Tokyo 143-8555 (JP)**

(74) Representative: **White, Duncan Rohan**
**Marks & Clerk LLP**
**Fletcher House (2nd Floor)**
**Heatley Road**
**The Oxford Science Park**
**Oxford OX4 4GE (GB)**

(54) **MICRONEEDLE ARRAY, AND MICRONEEDLE SHEET**

(57)    A microneedle array includes a plurality of hollow needles 11 and a plurality of individual liquid chambers 12 in communication with the hollow needles 11 that each are configured to hold a liquid composition to be ejected from tip end holes of the hollow needles 11. The length of each hollow needle 11 is 1 to 100 μm. The individual liquid chambers 12 are provided corresponding to the hollow needles 11.

FIG.1

(A)

(B)

EP 3 409 318 A1

**Description**

Field

The present invention relates to a microneedle array and a microneedle sheet.

Background

A microneedle array is a device having many minute needle-like protrusions arranged in an array.

Attempts have recently been made to use a microneedle array as a method of administering an ingredient of interest from a surface of a living body such as a skin into the body (transdermal administration). It is known that stratum corneum which is in a skin surface layer is an obstacle of delivery in transdermal administration, but microneedles penetrate through stratum corneum to enable delivery of the ingredient of interest under the stratum corneum.

Manufacturing methods, designs and materials of microneedle arrays suitable for such applications and techniques for carrying drugs have been developed.

As a method of carrying an ingredient of interest (for example, drug) to be administered on microneedles, mixing the ingredient and others in the material of a microneedle, applying a composition including the ingredient on the produced microneedle, and other methods are known.

For example, Patent Literature 1 discloses a technique using a microneedle array having two-stage microneedles, in which a large amount of drug is carried on the upper surface of a base portion and a pillar portion. Patent Literature 2 discloses an applicator for a microneedle array including a reservoir for liquid including a drug substance and a channel for transporting the liquid from the reservoir. Patent Literature 3 discloses a method of selectively and quantitively adding a necessary and sufficient amount of drug for a transdermal absorption sheet only to needle-like protrusions, in which the method involves forming a first polymer layer including a drug in needle-like recessed portions of a mold and applying and setting a second polymer solution not including a drug to form a polymer sheet including a stack of the first polymer layer and the second polymer layer, and removing the polymer sheet from the mold.

Minute needle-like protrusions forming a microneedle array can be deformed in some production methods, and the deformed protrusions may be bent or fractured when penetrating through skin.

For this problem, Patent Literature 4 discloses a method for blocking stress produced when a resin material is cooled to contract, in which microneedles are produced by presswork by producing a metal mold having a plate for blocking the stress.

A microneedle is a structure with a high aspect ratio and therefore requires complicated manufacturing steps and a special manufacturing device. In order to address this problem, Patent Literature 5 discloses a method in which an island-like etching mask having a thickness distribution is formed on a substrate, and the substrate is processed into needle shapes using the difference in etching rate between the etching mask and the substrate.

A microneedle array is known in which a needle has a through hole and the through hole is in communication with a microchannel. As a method of readily manufacturing a microneedle array having such a configuration, Patent Literature 6 discloses a configuration including a base for a microneedle array having microneedles each having a micro-through hole, a cover base bonded and fixed to the base for the microneedle array and having a structure for injecting a drug, and a micro-channel formed in the base for the microneedle array and the cover base to communicatively connect the structure for drug injection with the through holes of the microneedles.

Against a background of using intradermal infusion of a drug with microneedles that provides fast delivery of the drug without inducing pain as an alternative to hypodermic needles, Patent Literature 7 discloses a method of delivering a predetermined amount of fluid at a predetermined rate with microneedles with the length, the spacing, and the number thereof defined.

Unfortunately, microneedles that are long and penetrate through stratum corneum to reach dermis can be used only in medical practice. Moreover, forming a through hole in dermal tissue may cause skin irritation (erythema) or reduce skin moisture retention due to increase in moisture evaporation from skin.

Techniques of transdermal delivery of an ingredient of interest with microneedles are applied not only to medical products but also to cosmetics. In particular, fast-dissolving microneedles formed using a biodegradable resin as a material are suitable for delivery of cosmetic raw products and pharmaceutically active ingredients and are applicable to delivery of ingredients with poor water solubility and ingredients, such as polymers, difficult for transdermal absorption.

For example, Patent Literature 8 discloses, as a microneedle patch quickly absorbed in the body and a usage thereof, a method of quickly dissolving a microneedle array, in which moisture is supplied from the back surface of the microneedle array mainly formed of a water-swelling polymer and having a substrate with a thickness of 500 $\mu$m or less, and the moisture makes the microneedle array expand.

According to Patent Literature 8, the adequate length from the substrate of the microneedle to the needle tip end is from 100 $\mu$m to 800 $\mu$m. However, unless use conditions are designed as appropriate, a microneedle the length of which extends beyond corneum can reach dermis and its applications excluding medical practice are not suitable. Moreover, the dissolution rate of the biodegradable resin determines the rate of delivery.

Meanwhile, aspects having short microneedles and non-hollow protrusions have been proposed (see Patent Literatures 9 and 10).

Patent Literature 9 discloses a technique for administering a compound adhering to protrusions or included in protrusions through stratum corneum pressed to be thin, although the protrusions do not penetrate through stratum corneum of skin. The height of each protrusion is 10 $\mu$m to 3 mm.

Patent Literature 10 discloses an array having micro-protrusions capable of administering an active ingredient easily and without pain to skin through stratum corneum stretched to be thin, in which the micro-protrusions do not penetrate through stratum corneum of skin, preventing damage to stratum corneum. The height of the minute protrusion is 50 to 300 $\mu$m and has a particular shape. This structure can eliminate the possibility that the microneedle penetrates into dermis.

Summary

Technical Problem

In the application of a microneedle array to cosmetics or in order to supply an ingredient of interest without damaging dermal tissue, it is necessary that the needles or protrusions do not reach dermis.

However, as described above, some of the conventional microneedle arrays that have hollow needles penetrate into dermis because the needles are long. On the other hand, those having needles with a length reduced so as not to reach dermis or non-hollow protrusions carry a low dose of administration and therefore are unsatisfactory in terms of sustained releasability.

The present invention is then aimed to provide a microneedle array configured to carry a sufficient dose of administration without reaching dermis and with excellent sustained releasability.

Solution to Problem

In order to attain the aim, a microneedle array according to the present invention is a microneedle array that includes a plurality of hollow needles; and a plurality of individual liquid chambers in communication with the hollow needles that each are configured to hold a liquid composition to be ejected from tip end holes of the hollow needles, each of the hollow needles having a length of 1 to 100 $\mu$m, the individual liquid chambers being provided corresponding to the hollow needles.

Advantageous Effects of Invention

The present invention provides a microneedle array configured to carry a sufficient dose of administration without reaching dermis and with excellent sustained releasability.

Brief Description of Drawings

FIG. 1(A) is a schematic sectional view and FIG. 1(B) is a schematic perspective view of an exemplary microneedle array according to the present embodiment.
FIG. 2 is a schematic perspective view of an exemplary substrate for use in production of the microneedle array of the present embodiment.
FIG. 3 is a sectional diagram of the microneedle sheet of the present embodiment.
FIG. 4 is a schematic sectional view of an exemplary microneedle array of the present embodiment with a coating layer.

Description of Embodiments

A microneedle array and a microneedle sheet according to the present invention will be described below with reference to the figures. It should be noted that the present invention is not limited to the embodiments below and is susceptible to changes, such as other embodiments, addition, modification, and deletion, within a range that can be conceived by those skilled in the art, and any modes that achieve the operation effects of the present invention are to be encompassed in the range of the present invention.

[Microneedle Array]

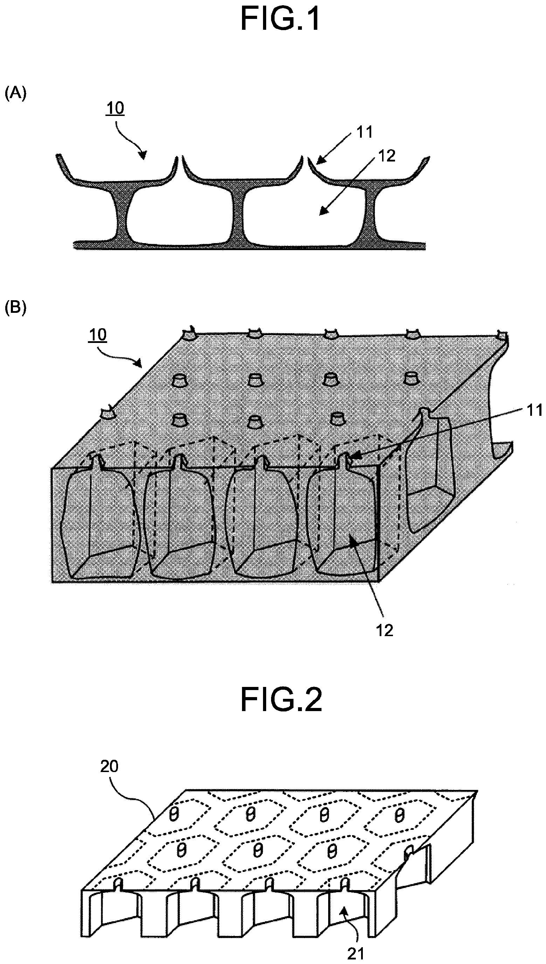

FIG. 1 is a diagram schematically illustrating a microneedle array of the present invention, in which (A) is a sectional view and (B) is a perspective view.

The microneedle array of the present invention includes a plurality of hollow needles 11 and a plurality of individual liquid chambers 12 in communication with the hollow needles 11 for holding a liquid composition to be ejected from the tip end holes of the hollow needles 11. The length of each hollow needle 11 is 1 to 200 $\mu$m, and the individual liquid chambers 12 are provided corresponding to the hollow needles 11.

The length of the hollow needle 11 is a length from the base to the tip end. If the hollow needle 11 is from 1 $\mu$m to 200 $\mu$m, when applied to skin, the hollow needle 11 can deliver a liquid composition from the tip end hole to stratum corneum without the tip end reaching dermis. If the length of the hollow needle 11 is less than 1 $\mu$m, transdermal delivery of a liquid composition cannot be performed well. The composition supplied to the skin surface or the vicinity of the surface is easily removed by washing skin or peeling.

If the length of the hollow needle 11 exceeds 200 $\mu$m, the hollow needle 11 is easily broken.

More preferably, the length of the hollow needle 11 is 4 to 20 $\mu$m.

The inner diameter of the tip end hole of the hollow needle 11 can be changed as appropriate to the extent that the liquid composition can be delivered to stratum corneum. Specifically, the inner diameter is preferably set to the extent that the hollow needle 11 inserted into skin does not pierce the inside of stratum corneum and the liquid composition is stably ejected. The inner diameter can be set to, but not limited to, for example, in a range of 2 to 20 $\mu$m.

The depth can be changed as appropriate by changing the pressing force against skin. The depth can be changed also by changing the area of the outer diameter and the inner diameter.

Considering that metabolization continuously occurs in stratum corneum of skin, and the composition supplied into stratum corneum is ejected to the outside of body for a relatively short time, the microneedle array is required to provide long-term supply and stable sustained releasability.

The microneedle array of the present embodiment can supply a liquid composition stably over a long time because an individual liquid chamber 12 capable of holding a sufficient amount of liquid composition is provided corresponding to each of the hollow needles 11 having a predetermined size. The microneedle array of the present embodiment therefore finds a wide variety of applications that require sustained release for a long term and can be used for application not only on skin but also in the agricultural field (for example, agricultural chemical sustained release device, administration to plant leaves) and as a variety of industrial materials and building materials.

All of a plurality of individual liquid chambers of the microneedle array may hold the same liquid composition, or the individual liquid chambers may hold different liquid compositions.

A conventional microneedle array having only a common liquid chamber is unable to apply a plurality of liquid compositions having different ingredients, which need to be mixed in order to be applied simultaneously. In mixing, it is often difficult to yield a homogenous mixture. For example, separation occurs due to the difference in specific gravity, or emulsification and other processes may be necessary. Even when homogeneous mixing is possible, a difference in the rate of absorption into skin can reduce the effect during application.

By contrast, the microneedle array of the present embodiment allows the individual liquid chambers to hold different liquid compositions and enables the use of different kinds of liquid compositions without mixing. The ratio of active ingredient blended may be minutely set for each individual liquid chamber. Furthermore, since the pitch of the microneedles is small, the compositions individually absorbed from skin can be homogenized in the body.

The thickness of the partition wall of the hollow needle 11 is preferably 0.01 to 5 $\mu$m.

The distance (pitch width) between adjacent hollow needles and the number of hollow needles per unit area can be selected as appropriate and can be changed according to the design of a mold used in manufacturing.

For example, the pitch of the hollow needles 11 is preferably 10 to 150 $\mu$m.

The number of the hollow needles 11 can be about 10,000 in an area of 5 cm$^2$, and 200 to 1,000 may be arranged in a length of 1 cm.

The capacity (in particular, height) of the individual liquid chamber 12 can be adjusted as appropriate depending on the conditions during manufacturing. For example, the capacity may be 1000 to 22500000 $\mu$m$^3$, and the height may be 10 to 1000 $\mu$m.

The thickness of the partition wall defining the individual liquid chambers 12 is preferably 0.1 to 5 $\mu$m.

The pitch of adjacent individual liquid chambers is preferably 10 to 150 $\mu$m with respect to the hollow needle 11.

The individual liquid chambers 12 may be shaped like a honeycomb. However, the embodiments are not limited thereto, and the sectional shape may be square or circular.

The amount of movement of the liquid composition to be held can be controlled according to the capacity of the individual liquid chamber 12. For example, the amount of movement is preferably 0.0005 mL/mm$^2$ to 0.003 mL/mm$^2$, preferably 0.001 mL/mm$^2$ to 0.002 mL/mm$^2$ in terms of processability.

If the amount of movement of the liquid composition is less than 0.0005 mL/mm$^2$, it is difficult to obtain satisfactory sustained releasability, and it is also difficult to supply the liquid composition to be held in the individual liquid chambers 12.

It is preferable that the microneedle array of the present embodiment does not contain metal as a constituent material. In the case of application to skin, the material forming the microneedle array is preferably a material having biocompatibility.

The material forming the microneedle array can be selected as appropriate as long as the material is at least plastically deformable, because a plastically deformable film is formed on a surface of a substrate for manufacturing and is expanded and stretched in a processing step. Examples of such a material include thermoplastic resins such as polycarbonate, polymer materials, ultraviolet-curable resins, and polydimethylsiloxane. A 30% dilute aqueous solution of gelatin may be used.

FIG. 2 illustrates an example of the substrate.

The method of manufacturing the microneedle array of the present embodiment involves arranging a material in the form of a film on a substrate 20 having a plurality of independent recessed portions 21 each having an opening, deforming the material under a reduced pressure to form a hollow structure, followed by setting and mold release.

In the step of affixing the material onto the substrate 20, pressing force is controlled so that the material will not come into contact with a portion serving as an opening and is brought into intimate contact only with a portion necessary for shape transfer.

The spatial portion expands under a reduced pressure and spreads within the material to form a hollow structure.

The liquid composition held in the individual liquid chambers 12 is not limited and can be selected from those containing a functional ingredient of interest and having flowability.

The functional ingredient can be selected according to the target to which the microneedle array of the present embodiment is applied. For example, in the case of cosmetics, the functional ingredient may be a cosmetic raw material, and in the case of medical products (nutrition drugs, medicines for diagnosis, and therapeutic drugs), the functional ingredient may be a pharmaceutically active ingredient.

Examples of the cosmetic raw material include whitening ingredients, such as ascorbic acid, vitamin C ethyl, vitamin C glycoside, ascorbyl palmitate, Kojic acid, Rucinol, tranexamic acid, licorice extract, vitamin A derivatives, and placenta extract; anti-wrinkle ingredients, such as retinol, retinoic acid, retinol acetate, retinol palmitate, EGF, cell culture extract, and acetylglucosamine; blood circulation-promoting ingredients, such as tocopherol acetate, capsain, and nonylic acid vanillylamide; diet ingredients, such as raspberry ketone, evening primrose extract, and seaweed extract; antibacterial ingredients, such as isopropylmethylphenol, photosensitizer, and zinc oxide; vitamins such as vitamin D2, vitamin D3, and vitamin K, and saccharides, such as glucose, trehalose, and maltose.

These ingredients can be used in the form of being encapsulated in a particle such as liposome or a polymer micelle or in the form of being immersed in a porous particle.

Examples of the pharmaceutically active ingredient, in particular, polymer pharmaceutically active ingredient, include biogenic peptides and derivatives thereof, nucleic acid, oligonucleotide, a variety of antigen proteins, bacteria, and virus fragments.

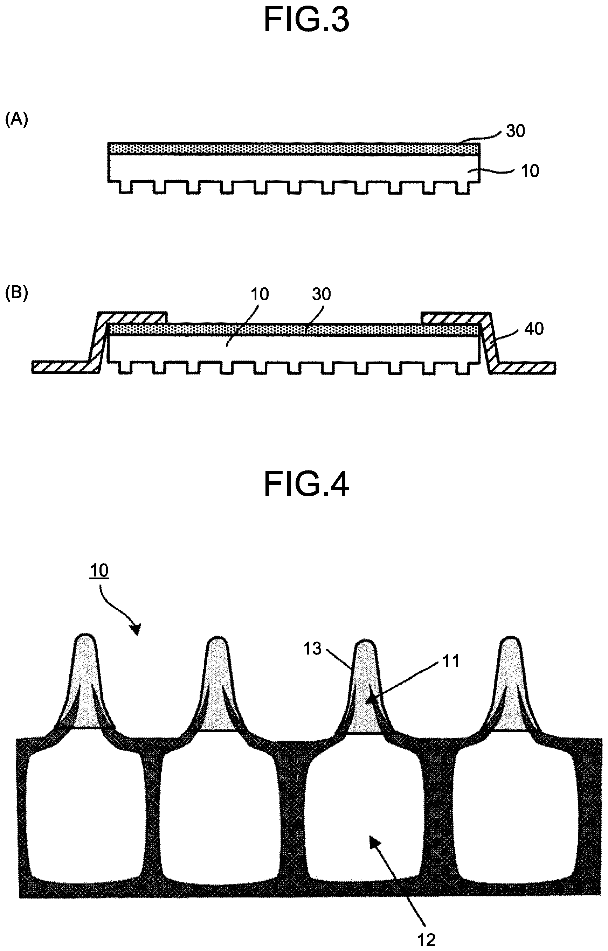

FIG. 4 illustrates a manner of the microneedle array of the present embodiment with a coating layer.

When the microneedle array is applied to skin, the hollow needle 11 may be buckled due to its low strength. By contrast, as illustrated in FIG. 4, the microneedle array 10 of the present embodiment has a coating layer 13 on an outer wall and/or an inner wall of a region including at least the tip end of the hollow needle 11.

The coating layer 13 at the tip end region of the hollow needle 11 as illustrated in FIG. 4 is preferably solid or may be gel.

Alternatively, the coating layer 13 may be formed in a filled state or may be like a sponge having pores formed by freeze drying during manufacturing. The sponge state enables control of the sustained releasability of a liquid held in individual liquid chambers 12. In this case, the tip end of the hollow needle 11 is impregnated with nonvolatile oil (for example, natural oil) for preventing leakage of the liquid.

A biocompatible polymer can be used as the material of the coating layer 13.

The biocompatible polymer may be a biodegradable polymer that is mild and less toxic to living body, has biocompatibility, and is dissolved and metabolized after being administered, or any resin that thermally melts. The biocompatible polymer can be selected as appropriate from polymers commonly used in medical applications.

The biodegradable polymer refers to a hydrolyzable polymer, and examples include trehalose, hyaluronic acid, sodium hyaluronate, dextran, chondroitin sulfuric acid, carboxymethyl cellulose, proteoglycan, collagen, gelatin, polylactide, polyglycolide, polycaprolactone, natural polymer, chitosan, cellulose, and polyvinyl alcohol.

The formed coating layer 13 is a thin film, and therefore any biodegradable material can be adapted.

The resin that thermally melts is a resin that undergoes a phase transition with temperature change and preferably becomes liquid, for example, under a condition of about 25°C to 30°C. Specifically, examples include thermosensitive synthetic polymers, such as poly(N-acryloylglycineamide)-co-poly(N-acetylacrylamide), poly(N-acryloylasparagineamide), and poly(allylamine)-co-poly(allyl urea).

Among those, the coating layer is preferably formed of a material that is not dissolved by the liquid held in the individual liquid chambers 12.

The coating layer 13 may be formed by any process and can be formed by a common process (for example, dipping, spray drying, etc.).

The coating layer 13 is formed by coating at least the tip end region of the hollow needle 11 with a solid material dissolving in the body (gelatin, water-soluble resin, etc.), and coating the other region with a variety of materials (polymer, hydrophobic resin, water-soluble resin, etc.).

As described above, the coating layer 13 is preferably formed of a material that is not dissolved by the liquid held in the individual liquid chamber 12. However, when the liquid in the individual liquid chamber 12 includes water and the coating is a water-soluble resin, the dissolution of the coating layer can be prevented by disposing a liquid that is not water-soluble (for example, oil, fat) or Vaseline on the solid material or encapsulating the water-soluble material in the form of a particle with a hydrophobic material.

When the coating layer 13 is formed of gelatin, gelatin serves as a cover during storage to achieve the effect of preventing leakage of the liquid in the individual liquid chamber 12 (anchor effect). When applied to skin, gelatin prevents buckling of the hollow needle 11 and facilitates insertion. The gelatin at the tip end region swells and dissolves in the body, so that the liquid in the individual liquid chamber 12 can flow to the tip end of the hollow needle 11 to be supplied.

The thickness of the coating layer 13 formed on the outer wall surface of the hollow needle 11 can be changed as appropriate to the extent that can prevent leakage of the liquid in the individual liquid chamber 12 as described above and achieves a strength with which the hollow needle 11 with the coating layer 13 is not bent when touching on the skin. The diameter of the outer periphery in a state in which the coating layer 13 is formed on the outer wall surface of the hollow needle 11 is preferably set as appropriate to the extent that achieves the effect of coating as described above and in which the coating layer 13 can be dissolved. The diameter of the outer periphery can be set, for example, but not limited to, in a range of 10 to 200 μm.

[Microneedle Sheet]

FIG. 3(A) and FIG. 3(B) are sectional schematic diagrams illustrating an exemplary microneedle sheet of the present invention having the microneedle array described above.

The microneedle sheet of the present embodiment includes a microneedle array 10 and a liquid composition containing a functional ingredient of interest held in the individual liquid chambers 12 and is affixed to skin in use. The liquid composition is supplied from a liquid composition supply material 30.

Alternatively, a microneedle sheet of the present embodiment includes a microneedle array 10 and an adhesive layer 40 for bonding the microneedle array to skin.

A specific example of the microneedle sheet is a cosmetic mask material.

The cosmetic mask material refers to a sheet-like material for covering skin with a non-woven fabric or a film containing a functional ingredient to allow an active ingredient to permeate into stratum corneum.

The cosmetic mask material having the microneedle array of the present embodiment includes an adhesive layer 40 for affixing to skin of the face or other parts. An example of the adhesive layer 40 is specifically an adhesive tape.

It is preferable that the adhesive tape has high water vapor permeability and excellent breathability. If the breathability is poor, moisture supply to skin is difficult to carry out, possibly causing skin roughness there.

Examples of the adhesive tape with high water vapor permeability and excellent breathability include a non-woven fabric, thin (10 μm or less) polyurethane, and a paper base material coated with a breathable adhesive. Alternatively, an adhesive sheet having a normal adhesive patterned on a sheet-like base material with high water vapor permeability may be used.

On the other hand, examples of the adhesive tape with low breathability include tapes with polyethylene, polyethylene terephthalate, nylon, and polypropylene base materials.

The liquid composition supply material 30 is deposited on the back surface of the microneedle array 10.

Specific examples of the liquid composition supply material 30 include gauze, non-woven fabric, tape, and aqueous gel mask materials. These materials containing a liquid composition (for example, skin lotion, serum, etc.) are brought into intimate contact with the back surface of the microneedle array 10 to supply the liquid composition to the individual liquid chambers 12.

The liquid composition supply material 30 preferably has a size that covers the entire back surface of the microneedle array 10, more preferably a size larger than the back surface in each direction.

The shape of the liquid composition supply material 30 is not limited and can be selected as appropriate depending on a target or a site that it is to be affixed to. Examples of the shape include circle, oval, rectangle, triangle, maqatama-like shape (curved comma shape), star shape, and desired shape depending on the place of application, such as a face mask-like shape.

The liquid composition may be delivered, for example, from an external supply source such as a syringe connected or other containers, using a tube or a luer connector. Alternatively, the microneedle sheet may be placed under a reduced pressure (vacuum) and supplied with a liquid composition to be delivered.

Examples

(Example 1)

A microneedle array having hollow needles and honeycomb-shaped individual liquid chambers in communication with the hollow needles is produced through a configuration and steps using the means below. The permeability and sustained releasability as well as skin irritation of the resultant microneedle array was evaluated.

<Configuration of Devices>

(1) Base

The base includes a honeycomb material for forming a honeycomb structure and a protective material.
The honeycomb material is a material that has flowability and ductility (not broken when formed into a thin film) in the process of deformation into a honeycomb shape and sets after being formed into a honeycomb shape. In this example, the honeycomb material is an energy beam-curable resin that cures in the ultraviolet region.
The protective material is a material to which the honeycomb material is applied and is used for protection so that gas will not escape in the honeycomb forming step (reducing pressure) and for protection from chipping by alleviating stress concentration in the removal step. Here, the protective material is preferably a material that allows ultraviolet rays to pass through, and examples include flexible plastic materials such as PET and PE.

(2) Template

The template includes a substrate and a cover, and the substrate and the cover are bonded to each other with adhesive.
The template has a shape for expanding the base (1) to form individual liquid chambers in a honeycomb shape and hollow needles.
The substrate has a structure that defines a surface shape and pitches and has openings in an inversely tapered shape. Although the material of the substrate used in the present example is nickel, a substrate made of a material such as silicon, stainless steel, and copper may be used.
The cover may be formed of the same material as the substrate. Although the material used in the present example is nickel, a material such as silicon, stainless steel, copper, iron, and glass may be used.
As the adhesive, for example, epoxy-based adhesive, acrylic adhesive, or thermoplastic (for example, polyurethane) adhesive may be used.

(3) Jig (Affixing Device)

The jig is a device for bringing the base (1) into intimate contact with the template (2).
Specifically, intimate contact is achieved by pressing with a roller member. The pressing force by the jig is set to be smaller than the pressing force by the pressing device to be used in the next step.

(4) Pressing Device

The pressing device is a device for generating a desired pressure uniformly on the base (1) and the template (2). The pressing by the pressing device deforms the base to control the shape transfer to the template. The pressing is performed under atmospheric pressure and may be performed in combination with the jig (3).

(5) Decompression Device

The decomposition device is a device for reducing the pressure in the environment of the base (1) and the template (2) (making a vacuum state). Reducing the pressure forms a honeycomb shape and hollow needles in the honeycomb material.

(6) Curing Device (Ultraviolet Ray Emitting Device)

The curing device in this example is a device emitting ultraviolet rays. After the honeycomb shape and the hollow needles are formed in the base (1), ultraviolet rays are applied for curing.

(7) Removing Device (or Jig)

The removing device is a device for removing, from the template (2), the base having the honeycomb shape and the hollow needles in a microneedle array structure. In this example, a forcipate-shaped jig is used to pinch the base and pull up the base for removal.

<Manufacturing Process>

i) Application Step (Affixing Step)

The base formed of the honeycomb material applied on the protective material in advance is affixed to the template with the affixing device. Pressure control is performed so that the honeycomb material will not enter the openings of the template more than necessary. The base is affixed from the end portion thereof so that bubbles will not be trapped in the other portions.

ii) Transfer Step

The pressing device pushes the base against the template with a uniform pressure, so that the shape of the template is transferred to a desired place.
In this example, the pressing force was 60 kPa.

iii) Honeycomb Forming Step

The decompression device reduces the pressure inside the container (environment) including the base and the template, so that a relative pressure difference is generated, and the gas in the recessed portions (spaces) of the template expands to enter the inside of the honeycomb material of the base. On the other hand, since the honeycomb material at a portion in intimate contact with the template does not flow, independent cavities are formed to serve as the honeycomb shape and the hollow needles.
In this example, the pressure reduction time was 90 seconds.

iv) Curing Step

The curing device emits ultraviolet rays to cure the material having the honeycomb shape and the hollow needles.

v) Removal Step

The base having the honeycomb shape and the hollow needles in the form of a microneedle array structure is removed with the removing device.
In the microneedle array obtained through the steps above, the length of the hollow needle was 7 $\mu$m, the inner diameter of the tip end hole was 6.2 $\mu$m, and the capacity of each individual liquid chamber was 2 mm$^3$.

<Permeability and Sustained Releasability Evaluation>

A microneedle sheet having a liquid composition supply material affixed to the back surface of the microneedle array was fabricated, and the permeability and the sustained releasability of the liquid composition to a target were evaluated.
A sheet (10 cm by 10 cm) sufficiently impregnated with a liquid composition including a labeled substance (calcein) was used as the liquid composition supply material.
The resultant microneedle sheet was affixed to agarose gel and left for 30 minutes under a temperature condition of 20°C.
The cross section of the agarose gel was observed, and the moving distance of calcein from the tip end of the microneedle array was measured and evaluated according to the criteria below. Table 1 shows the results.

Evaluation Criteria

A: The moving distance is 0.20 mm or less.
B: The moving distance is 0.15 mm or less.
C: The moving distance is 0.10 mm or less.
D: The moving distance is 0.05 mm or less.
E: The moving distance exceeds 0.20 mm.

Among the evaluation criteria above, Grades A to D are suitable for the application as a cosmetic product, and in terms of permeability and sustained releasability, Grade A is most excellent (excellent in the order of A>B>C>D).

<Skin Irritation Evaluation>

The microneedle array was affixed to skin of the back of the hand of each of 15 subjects for evaluation. The response in terms of irritation after the elapse of 30 seconds was evaluated based on the criteria below. The number of people who made evaluations was counted, and the proportion (%) was calculated. Table 1 shows the results.

Evaluation Criteria

◎: feel no irritation or discomfort
○: feel slight discomfort
△: feel slight pain
×: feel pain and discomfort

Table 1

| | | Example 1 | Example 2 | Example 3 | Example 4 | Example 5 | Example 6 | Example 7 | Example 8 | Example 9 | Comparative Example 1 |
|---|---|---|---|---|---|---|---|---|---|---|---|
| Conditions | Pressing force (kPa) | 60 | 20 | * | * | * | * | * | 60 | 60 | - |
| | Pressure reduction time (sec) | 90 | 90 | * | * | * | * | * | 90 | 90 | - |
| Shape | Length of hollow needle (pm) | 7 | 1 | 100 | 10 | 3 | 20 | 47 | 7 | 7 | 300 |
| | Inner diameter of tip end hole (pm) | 6.2 | 5 | 20 | 5 | 4 | 5 | 10 | 6.2 | 6.2 | 10 |
| | Capacity of individual liquid chamber ($mm^3$) | 2 | 2 | 2 | 2 | 0.5 | 20 | 25 | 2 | 2 | - |
| Evaluation | Labelled substance for use in permeability and sustained releasability evaluation | calcein | calcein | calcein | calcein | calcein | calcein | calcein | calcein encapsulated in block polymer | calcein encapsulated in liposome | calcein |
| | Permeability and sustained releasability | B | C | C | B | D | A | A | B | B | E |
| | Skin irritation ◎ | 86.7% | 100.0% | 33.3% | 86.7% | 93.3% | 73.3% | 53.3% | - | - | 0.0% |
| | ○ | 13.3% | 0.0% | 60.0% | 13.3% | 6.7% | 20.0% | 26.7% | - | - | 0.0% |
| | △ | 0.0% | 0.0% | 6.7% | 0.0% | 0.0% | 6.7% | 20.0% | - | - | 13.3% |
| | × | 0.0% | 0.0% | 0.0% | 0.0% | 0.0% | 0.0% | 0.0% | - | - | 86.7% |

\* value adjusted as appropriate

EP 3 409 318 A1

It has been found that the microneedle array in this example had a hollow needle with a length of 7 $\mu$m and therefore would not reach dermis when applied to skin, and the result of skin irritation evaluation has revealed that the microneedle array would not give pain. It has been also found that the capacity of each individual liquid chamber is 2 mm$^3$, which is enough to carry a dose of administration, and the permeability and the sustained releasability were excellent.

(Example 2)

A microneedle array was fabricated in the same manner as in Example 1 except that the pressing force in the transfer step was 20 kPa, and the permeability and the sustained releasability as well as the skin irritation was evaluated.
In the resultant microneedle array, the length of the hollow needle was 1 $\mu$m, the inner diameter of the tip end hole was 5 $\mu$m, and the capacity of each individual liquid chamber was 2 mm$^3$.
The permeability and the sustained releasability were evaluated as suitable for the application as a cosmetic product. For the evaluation of skin irritation, all the subjects felt no discomfort.

(Example 3)

A microneedle array having a hollow needle with a length of 100 $\mu$m, a tip end hole with an inner diameter of 20 $\mu$m, and each individual liquid chamber with a capacity of 2 mm$^3$ was fabricated by adjusting the pressing force and the pressure reduction time in the transfer step. The permeability and the sustained releasability as well as the skin irritation were evaluated.
The permeability and the sustained releasability were evaluated as suitable for the application as a cosmetic product, and the skin irritation was also evaluated as satisfactory.

(Example 4)

Silicone rubber (PDMS) with high gas permeability was used as the material of the template.
The honeycomb material was affixed to the template with low pressure and then pushed against the template with a uniform pressure to transfer the portion other than the tip end portions.
In the honeycomb forming step, gas absorbed into the template was emitted to enter the honeycomb material and expand to form a honeycomb shape.
The other steps and conditions were similar to those in Example 1 to fabricate a microneedle array.
In the resultant microneedle array, the length of the hollow needle was 10 $\mu$m, the inner diameter of the tip end hole was 5 $\mu$m, and the capacity of each individual liquid chamber was 2 mm$^3$.
The permeability and the sustained releasability were evaluated as suitable for the application as a cosmetic product, and the skin irritation was also evaluated as satisfactory.

(Example 5)

A microneedle array was fabricated in the same manner as in Example 1 except that the pressing time and the pressure reduction time were adjusted so that the length of the hollow needle of the microneedle array would be 3 $\mu$m, the inner diameter of the tip end hole was 4 $\mu$m, and the capacity of each individual liquid chamber was 0.5 mm$^3$, and the permeability and the sustained releasability as well as the skin irritation was evaluated.
The permeability and the sustained releasability were slightly inferior but evaluated as suitable for the application as a cosmetic product, and the skin irritation was also evaluated as satisfactory.

(Example 6)

A microneedle array was fabricated in the same manner as in Example 1 except that the pressing time and the pressure reduction time were adjusted so that the length of the hollow needle of the microneedle array would be 20 $\mu$m, the inner diameter of the tip end hole was 5 $\mu$m, and the capacity of each individual liquid chamber was 20 mm$^3$. The permeability and the sustained releasability as well as the skin irritation was evaluated.
The permeability and the sustained releasability were significantly excellent, and the skin irritation was also evaluated as satisfactory.

(Example 7)

A microneedle array was fabricated in the same manner as in Example 1 except that the pressing time and the pressure reduction time were adjusted so that the length of the hollow needle of the microneedle array would be 47 $\mu$m, the inner diameter of the tip end hole would be 10 $\mu$m, and the capacity of each individual liquid chamber would be 25 mm$^3$. The permeability and the sustained releasability as well as the skin irritation were evaluated.

It has been found that the permeability and the sustained releasability were significantly excellent. For the evaluation results of skin irritation, 20% made an evaluation as $\Delta$ but 0% evaluated made an evaluation as $\times$.

(Example 8)

In the permeability and sustained releasability evaluation, a microneedle array was fabricated in the same manner as in Example 1 for evaluation, except that calcein serving as a labeled substance was encapsulated in a block polymer (methoxypoly(ethylene glycol)-block-poly(lactide-co-glycolide 200Da-15000Da (manufactured by Sigma-Aldrich Co. LLC) as a block copolymer).

Even with different liquid compositions, the permeability and the sustained releasability were satisfactory with no problem.

(Example 9)

In the permeability and sustained releasability evaluation, a microneedle array was fabricated in the same manner as in Example 1 for evaluation, except that calcein serving as a labeled substance was encapsulated in liposome.

Even with different liquid compositions, the permeability and the sustained releasability were satisfactory with no problem.

(Comparative Example 1)

A microneedle array was fabricated by forming protrusions using a medical grade polycarbonate by heat cycle ejection molding, followed by hollowing.

In the resultant microneedle array, the length of the hollow needle was 300 $\mu$m and the inner diameter of the tip end hole was 10 $\mu$m. No individual liquid chambers were formed.

Since no individual liquid chambers were formed, the permeability and the sustained releasability did not satisfy the standards required for application as a cosmetic product. For evaluation of skin irritation, 14 out of 15 subjects (86.7%) felt pain and discomfort.

(Example 10)

<Formation of Coating Layer>

On a microneedle array obtained in the same manner as in Example 1, a coating layer to cover the outer wall and the inner wall of a region including the tip end of a hollow needle was formed with a biodegradable polymer (trehalose).

The diameter (outer diameter) of the outer periphery of the coating layer formed on the outside of the hollow needle was 100 $\mu$m.

The formed coating layer was a filled solid.

<Buckling Ratio>

The microneedle array was affixed to skin of the back of the hand of a subject for evaluation. The state of the hollow needle after one hour was observed with a scanning microscope.

The number (X) of deformed or bent needles of 50 hollow needles was counted, and the buckling ratio was obtained according to the equation below. Table 2 shows the results.

$$\text{Buckling ratio}(\%) = (X/50) \times 100$$

<Solubility of Coating Layer>

A microneedle sheet was fabricated by affixing a liquid composition supply material to the back surface of the microneedle array having a coating layer.

A sheet (10 cm by 10 cm) sufficiently impregnated with a liquid composition including a labeled substance (calcein) was used as a liquid composition supply material.

The resultant microneedle sheet was soaked in a physiological saline solution, and calcein in the physiological saline solution was detected with a highspeed liquid chromatography mass spectrometer (LCMS). The timing when calcein was detected was considered as the timing when the coating layer was dissolved, and the time taken for detection was evaluated based on the criteria below. Table 2 shows the results.

Evaluation Criteria

    A: shorter than 0.5 minute
    B: shorter than 1 minute
    C: shorter than 2 minutes
    D: 5 minutes or longer

In the above evaluation criteria, the solubility is excellent in the order of A>B>C>D. Whatever of the above evaluation criteria may be, the application to a cosmetic product is possible. In terms of practical use, evaluation A is suitable.

Table 2

| | | | Example 10 | Example 11 | Example 12 | Example 13 | Example 14 | Comparative Example 2 | Comparative Example 3 |
|---|---|---|---|---|---|---|---|---|---|
| Conditions | | Pressing force (kPa) | 60 | 60 | 60 | 60 | 60 | 60 | - |
| | | Pressure reduction time (sec) | 90 | 90 | 90 | 90 | 90 | 90 | - |
| Shape | | Length of hollow needle (pm) | 7 | 7 | 7 | 7 | 7 | 7 | 200 |
| | | Inner diameter of tip end hole ($\mu$m) | 6.2 | 6.2 | 6.2 | 6.2 | 6.2 | 6.2 | 50 |
| | | Capacity of individual liquid chamber (mm$^3$) | 2 | 2 | 2 | 2 | 2 | 2 | - |
| | | Coating layer outer diameter ($\mu$m) | 100 | 200 | 50 | 300 | 100 | - | - |
| | | Coating layer state | filled | filled | filled | filled | porous | - | - |
| Evaluation | | Labelled substance for use in permeability and sustained releasability evaluation | calcein | calcein | calcein | calcein | calcein | calcein | calcein |
| | | Buckling ratio (%) | 10 | 2 | 70 | 15 | 15 | 95 | 76 |
| | | Solubility (dissolving rate) | B | C | A | D | A | A | D |

EP 3 409 318 A1

(Examples 11 to 13)

A microneedle array and a microneedle sheet were produced in the same manner as in Example 10, except that the outer diameter of the coating layer formed on the outside of the hollow needle was set to a value shown in Table 2, and the buckling ratio and the solubility of the coating layer were evaluated. Table 2 shows the results.

(Example 14)

A microneedle array and a microneedle sheet were produced in the same manner as in Example 10, except that the coating layer formed on the outside of the hollow needle was porous, and the buckling ratio and the solubility of the coating layer were evaluated. Table 2 shows the results.

(Comparative Example 2)

For a microneedle array and a microneedle sheet in Example 1 without a coating layer on the outside of the hollow needle, the buckling ratio and the solubility of the coating layer were evaluated in the same manner as in Example 10. Table 2 shows the results.

(Comparative Example 3)

For a microneedle array and a microneedle sheet in Comparative Example 1 without a coating layer on the outside of the hollow needle, the buckling ratio and the solubility of the coating layer were evaluated in the same manner as in Example 10. Table 2 shows the results.

Reference Signs List

10　　microneedle array
11　　hollow needle
12　　individual liquid chamber
13　　coating layer
20　　substrate
21　　recessed portion
30　　liquid composition supply material
40　　adhesive layer

Citation List

Patent Literature

Patent Literature 1: Japanese Patent Application Laid-open No. 2015-109963
Patent Literature 2: Japanese Patent No. 5553612
Patent Literature 3: Japanese Patent Application Laid-open No. 2010-069253
Patent Literature 4: Japanese Patent Application Laid-open No. 2010-247535
Patent Literature 5: Japanese Patent Application Laid-open No. 2008-296037
Patent Literature 6: Japanese Patent Application Laid-open No. 2011-078654
Patent Literature 7: Japanese Unexamined Patent Application Publication (Translation of PCT Application) No. 2012-509106
Patent Literature 8: Japanese Patent Application Laid-open No. 2013-075165
Patent Literature 9: Japanese Patent Application Laid-open No. 2007-089792
Patent Literature 10: Japanese Patent No. 5597254

**Claims**

1. A microneedle array comprising:

   a plurality of hollow needles; and
   a plurality of individual liquid chambers in communication with the hollow needles that each are configured to hold a liquid composition to be ejected from tip end holes of the hollow needles, each of the hollow needles having a length of 1 to 100 $\mu$m, the individual liquid chambers being provided corresponding to the hollow needles.

2. The microneedle array according to claim 1, wherein the microneedle array does not contain metal as a constituent material.

3. The microneedle array according to claim 1 or 2, wherein the microneedle array includes a coating layer on an outer wall and/or an inner wall of a region including at least a tip end portion of the hollow needle.

4. The microneedle array according to claim 3, wherein the coating layer is formed of a biocompatible polymer.

5. A microneedle sheet comprising:

   the microneedle array of any one of claims 1 to 4; and
   a liquid composition containing a functional ingredient of interest held in the individual liquid chambers, wherein the microneedle sheet is used so as to be affixed to skin.

6. A microneedle sheet comprising:

   the microneedle array of any one of claims 1 to 4 and
   an adhesive layer for bonding the microneedle array to skin.

# FIG.1

(A)

(B)

# FIG.2

# FIG.3

(A)

30

10

(B)

10    30

40

# FIG.4

10

13

11

12

## INTERNATIONAL SEARCH REPORT

International application No.

PCT/JP2017/001386

### A. CLASSIFICATION OF SUBJECT MATTER
*A61M37/00*(2006.01)i

According to International Patent Classification (IPC) or to both national classification and IPC

### B. FIELDS SEARCHED

Minimum documentation searched (classification system followed by classification symbols)
A61M37/00

Documentation searched other than minimum documentation to the extent that such documents are included in the fields searched
| | | | |
|---|---|---|---|
| Jitsuyo Shinan Koho | 1922–1996 | Jitsuyo Shinan Toroku Koho | 1996–2017 |
| Kokai Jitsuyo Shinan Koho | 1971–2017 | Toroku Jitsuyo Shinan Koho | 1994–2017 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| Y | WO 2015/024816 A1 (LTS LOHMANN THERAPIE-SYSTEME AG), 26 February 2015 (26.02.2015), page 2, line 9 to page 6, line 3; fig. 1 to 4 & US 2016/0158515 A1 paragraphs [0016] to [0031]; fig. 1 to 4 & JP 2016-527999 A & EP 2839853 A1 & CA 2914993 A1 & CN 105473172 A | 1-6 |
| Y | JP 2007-532245 A (Allergan, Inc.), 15 November 2007 (15.11.2007), paragraphs [0075] to [0080]; fig. 17 to 18 & US 2010/0256594 A1 paragraphs [0096] to [0101]; fig. 17 to 18 & JP 4981660 B2 & WO 2005/110525 A2 & EP 3108925 A2 & CA 2562643 A1 | 1-6 |

☒ Further documents are listed in the continuation of Box C.  ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 31 March 2017 (31.03.17) | 18 April 2017 (18.04.17) |

| Name and mailing address of the ISA/ Japan Patent Office 3-4-3,Kasumigaseki,Chiyoda-ku, Tokyo 100-8915,Japan | Authorized officer Telephone No. |
|---|---|

Form PCT/ISA/210 (second sheet) (January 2015)

**INTERNATIONAL SEARCH REPORT**

International application No.

PCT/JP2017/001386

C (Continuation). DOCUMENTS CONSIDERED TO BE RELEVANT

| Category* | Citation of document, with indication, where appropriate, of the relevant passages | Relevant to claim No. |
|---|---|---|
| Y | US 6565532 B1 (THE PROCTER & GAMBLE CO.), 20 May 2003 (20.05.2003), column 44, line 27 to column 46, line 48; fig. 58, 60 to 61 & WO 01/91846 A2 | 1-6 |
| Y | JP 2013-90808 A (Toppan Printing Co., Ltd.), 16 May 2013 (16.05.2013), paragraphs [0044] to [0048]; fig. 6 (Family: none) | 3-4 |
| A | JP 2004-503341 A (Becton, Dickinson and Co.), 05 February 2004 (05.02.2004), paragraphs [0014] to [0037]; fig. 1 to 9 & US 6656147 B1 column 4, line 20 to column 9, line 45; fig. 1 to 9 & JP 4815095 B2 & WO 02/05889 A1 | 1-6 |
| A | JP 2012-105791 A (ASTI Corp.), 07 June 2012 (07.06.2012), paragraphs [0033] to [0036]; fig. 10 (Family: none) | 1-6 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2015109963 A
- JP 5553612 B
- JP 2010069253 A
- JP 2010247535 A
- JP 2008296037 A
- JP 2011078654 A
- JP 2012509106 W
- JP 2013075165 A
- JP 2007089792 A
- JP 5597254 B